# EUROPEAN PATENT APPLICATION

(11) **EP 1 949 792 A2**
(43) Date of publication of application: **30.07.2008**
(21) Application number: 07013597.5
(22) Date of filing: 11.07.2007
(51) Int. Cl.: A21D 13/08, A23G 3/48, A23G 4/06, A23L 1/30, A23L 2/52, A61K 36/81, A61P 3/04

(54) **The healthy and functional foods for the obesity patients using purple-colored potato**

(30) Priority: 17.01.2007 KR 20070005435
(71) Applicant: Potato Valley Co., Ltd., Hongcheon-eup Hongcheon-gun Kangwon do (KR)
(72) Inventor: Lim, Hak Tae, Toegye-dong, Chuncheon-si, Kangwon-do 200-170 (KR); Kim, Youn Soo, Toegye-dong, Chuncheon-si, Kangwon-do 200-170 (KR); Kim, Sung Hoon, Dogok-dong, Kangnam-gu, Seoul 135-270 (KR); Rhee, Yun Hee, Kangam-gu, Seoul 135-120 (KR); Lee, Min Ho, Suji-gu, Yongin-si, Gyeonggi-do 448-130 (KR)
(74) Representative: Hiebl, Inge Elisabeth

(57) **Abstract**

Disclosed is a novel healthy and functional food having an obesity-suppressing activity. The healthy and functional food according to the present invention is manufactured by using an extract or a raw juice of purple-colored potato as a main/minor ingredient, or by adding the extract or a raw juice of purple-colored potato to various favorite foods. The extract of purple-colored potato according to the present invention has an obesity-suppressing activity by preventing an adipose cell from being differentiated and reducing leptin protein aiding to differentiate into adipose cells, and has an ability to reduce free fatty acid which is a hyperlipidemia-inducing factor and cholesterol in blood. Therefore, the healthy and functional food containing the extract of purple-colored potato according to the present invention can be useful to treat obesity patients including a wide age group of teenagers and adults and old persons.

## Description

### BACKGROUND OF THE INVENTION

### 1. Field of the Invention

The present invention relates to a healthy and functional food containing a novel species purple-colored potato *(Solanum tuberosum* L., cv. Bora valley) extract and having an obesity-suppressing activity.

### 2. Description of the Prior Art

In recent years, there has been an increasing population of overweight and obese persons due to the improved eating habits and reduced living activities with the economic growth and changes in industrial infrastructures.

The obesity has appeared as a big social problem in these days. The fatness used to be a symbol of rich people's life style, but it has recently revealed that the obesity is a major cause of geriatric diseases such as diabetes mellitus, arteriosclerosis, heart diseases, etc., which is a big fear rather than the arrogance. For that reason, there is an important dispute for health cares.

The obesity refers to a state where fat is excessively accumulated in the body, that is, a state where the fat accounts for a relatively high amount in the body weight. If a man has an adipose cell of 20-50 % or more and a woman has an adipose cell of 30-35 % or more, based on the total body weight, they are considered to be obese. Generally, the obesity can be divided into (1) a light obesity if a body weight is within a range of 10-20 % of the standard body weight, (2) a middle obesity if a body weight is within a range of 20-50 % of the standard body weight, and (3) a morbid obesity if a body weight exceeds 50 %.

The obesity is classified into simple obesity and symptomatic obesity according to its cause, and also classified into hypertrophic obesity, hyperplastic obesity and mixed obesity in the other cases, or central obesity in which a fat is excessively accumulated in an abdominal region; and peripheral obesity in which a hip, a thigh and a shoulder, depending on the fat distribution. The central obesity is called if a man has a waist-hip ratio (WHR) of 1.0 or more and a woman has a waist-hip ratio (WHR) of 0.9 or more. An increased fat in internal organs around the center of abdomen becomes breeding grounds for a variety of geriatric diseases even though one is light weight.

However, these symptoms of the obesity generally results in development of adipose fat, and therefore suppressing growth of the adipose cells may be the most effective method for capable of suppressing the obesity ultimately.

Fat cells function to control the growth and differentiation of own cells, and also maintain energy homeostasis in the body. The adipose fat, which has been considered to be a simple energy storage tissue for a long time, has become an important research subject due to its role in the energy balance and metabolic diseases including obesity. The obesity caused by the excessive differentiation of fat cells and the excessive supply of unbalanced energy is prescribed as one disease rather than the simple appearance problem by the WHO in recent years. In particular, much attention has been paid to the differentiation of fat cells since the obesity acts as the most important risk factor to induce adult diseases such as hypertension, hyperlipidemia, arteriosclerosis, cardiac disorder and non-insulin dependent diabetes mellitus (NIDDM).

The liver, which is an organ that plays a critical role in the lipid metabolism, is a site for synthesis and oxidation of fatty acid, and for synthesis of triglyceride, phospholipid, cholesterol and lipoproteinl. In particular, the triglyceride may be accumulated in the liver due to the various unknown reasons, but the excessive accumulation of the triglyceride is considered to be a morbid state. At this time, if the triglyceride is chronically accumulated in the liver, the fibrosis in the liver cells gradually develops into hepatocirrhosis and abnormal liver function.

There are many causes of the fatty liver, but the causes of the fatty liver may be divided into two main reasons. Adipokinesis from the adipose fat or hydrolysis of lipoprotein or chylomicron triacylglycerol in the other tissues rather than the liver are associated with the increased level of free fatty acid in blood plasma. Eventually, after the free fatty acid is absorbed into the liver, then the triglyceride is accumulated in the liver if the production of lipoproteins in serum does not surpass the inflow of the fatty acid. Second, a normal lipid metabolism in the liver suppresses synthesis of proteins, for example synthesis of apoprotein or lipoprotein required for transportation from the liver to other tissues by means of toxic substances, etc., resulting in the accumulation of the fat in the liver. Such accumulation of the fat in the liver causes abnormal functions and morphological changes of the liver tissue, and therefore the accumulated fat functions a factor that has an adverse effect on the normal lipid metabolism in the liver which is an important organ of main metabolisms such as energy supplies into the body, etc.

The conventional supplementary food groups used for treating obese diseases are in a solid or liquid form and serve to reduce moisture inducing diarrhea in the body or remove coprostasis with colon irrigation, but does not take part in the formation and differentiation of the adipose fat. In the case of the morbid obesity, chemically synthesized drugs other than the natural substances are also administered, but they have many problems of side effects in the body.

Potato has a short cultivation period and a relatively high productivity per unit area and is strongly adapted to the external environments, and therefore it is one of the four food crops next to the rice, wheat and corn, and they are cultivated in about 130 countries all over the world. Potato has a lower calory than the rice and wheat, and is nearly close to vegetables in the aspect of the content of nutrients such as vitamin C, B₁, B₂, niacin, etc. Accordingly, there have been many attempts to perform a general sitological analysis and anti-oxidant activity of the potato.

In recent years, a war for the good plant seeds becomes more severe due to the change in the international agricultural environments, and the foods may become weaponry in the near future. Therefore, every country do the best for protecting plant variety rights related to the food crops. In this state of things, the inventors developed many varieties of novel functional potato species and registered them as nationally recognized potato varieties (National Seed Management Office, Ministry of Agriculture, Republic of Korea).

As filed by the inventors, Korean Patent Registration No. 10-628427 (1) discloses a potato extract having growth-inhibiting and promoting effects on human intestinal bacteria extracted from valley potato (Bora Valley, Juice Valley, Dasom Valley, Gogu Valley, etc.) cultivars and a functional food using the same, Korean Patent Registration No. 10-654231 (2) discloses a thermostable peptide isolated from *Solanum tuberosum* L cv. Gogu Valley, and Korean Patent Registration No. 10-654232 (3) discloses a thermostable peptide isolated from *Solanum tuberosum* L cv. Golden Valley and functional resources use of the same, and Korean Patent Publication No. 10-2005-110186 (4) discloses anti-oxidative compounds extracted from potato *(Solanum tuberosum* L., cv Bora valley).

The purple-colored potato *(Solanum tuberosum* L., cv Bora valley), according to the present invention, is a potato having a purple-colored inner and outer parts developed by the inventors, and a novel potato species for processing a purple-colored potato chip, which was filed with the Republic of Korea National Seed Management Office for Plant Variety Protection (Applicaton No. 2002-11) and registered as a national potato variety (Registration No. 1-5-2004-5) for chipping on 2004. The true potato seeds were obtained by an artificial, traditional crossing method, rather than a genetically engineered method, in which A87spx14-4 was used as a mother parent and gurmeys purple was used as a father parent. Throughout the several years of selection process started from seedling stage, one elite clone was finally selected and substantially tested for the productivity and local adaptability in three major potato production areas in South Korea (Seo-myeon, Chunchon city; KwangHwal-myeon, Kimje city; HwaengGhe, Daegwanryong, etc.) from 1999 to 2001.

Application of the potato variety registration was filed on 2002, and further tested by 3 national seed research institutes for two years (2002 to 2003) under the request of the Korea National Seed Management Office, and then finally acknowledged as for the superiority of the novel potato species, especially for processing a purple-colored potato chip for the first time in Korea on 2004, and it was named as "Bora valley". The inventors also obtained the Korean Patent (No. 10-2005-110186) on 2006, disclosing high levels of anti-oxidative compounds in potato *(Solanum tuberosum* L., cv Bora valley).

The inventors attempted further studies on the novel potato species of "Bora valley" and found that an ethanol extract of purple-colored potato may be used as a healthy and functional food for treating the obesity patients since the ethanol extract serves to suppress the growth of adipose cells and the differentiation of pre-adipose cells into adipose cells and control an amount of expressed leptin transmitted to the pituitary gland. Therefore, the present invention was completed based on the above facts.

### SUMMARY OF THE INVENTION

Accordingly, the present invention is designed to solve such drawbacks of the prior art, and therefore an object of the present invention is to provide a healthy and functional food containing a novel species purple-colored potato.

One embodiment of the present invention is achieved by providing a healthy and functional food for treating obesity patients, the food containing an extract or raw juice, or dry power of purple-colored potato (*Solanum tuberosum* L*.* cv. Bora valley).

### BRIEF DESCRIPTION OF THE DRAWINGS

These and/or other aspects and advantages of the invention will become apparent and more readily appreciated from the following description of the preferred embodiments, taken in conjunction with the accompanying drawings.

FIG. 1 a is a photographic diagram showing whether the induction of adipose cells is suppressed in mice treated with the ethanol extract of the purple-colored potato at an increasing concentration by using an Oil-red O staining method.

FIG. 1b is a graph illustrating absorbance of the adipose cells dissolved in isopropanol after the induction of adipose cells is suppressed in mice treated with the ethanol extract of the purple-colored potato at an increasing concentration by using an Oil-red O staining method.

FIG. 2 is a graph illustrating the content of leptin in the adipose cells after mice were treated with the ethanol extract of the purple-colored potato at an increasing concentration.

FIG. 3 is a graph illustrating the weights of visceral fat, liver and kidney in the ethanol diet group of the purple-colored potato.

FIG. 4a is a tomograph taken from abdomens mice in the normal diet group using a magnetic resonance imaging system.

FIG. 4b is a tomograph taken from abdomens mice in the high fat diet group using a magnetic resonance imaging system.

FIG. 4c is a tomograph taken from abdomens mice treated with 100mg/kg ethanol extract of the purple-colored potato in the high fat diet group using a magnetic resonance imaging system.

FIG. 4d is a tomograph taken from abdomens mice treated with 500mg/kg ethanol extract of the purple-colored potato in the high fat diet group using a magnetic resonance imaging system.

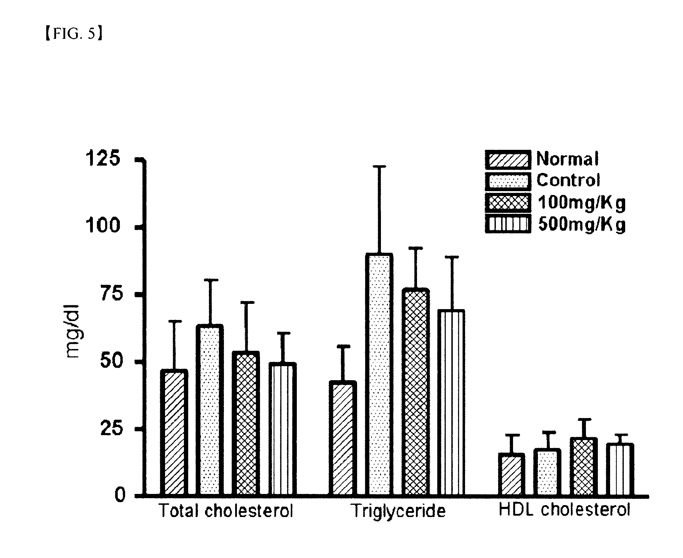

FIG. 5 is a graph illustrating the content of lipid in blood in an ethanol extract diet group of the purple-colored potato.

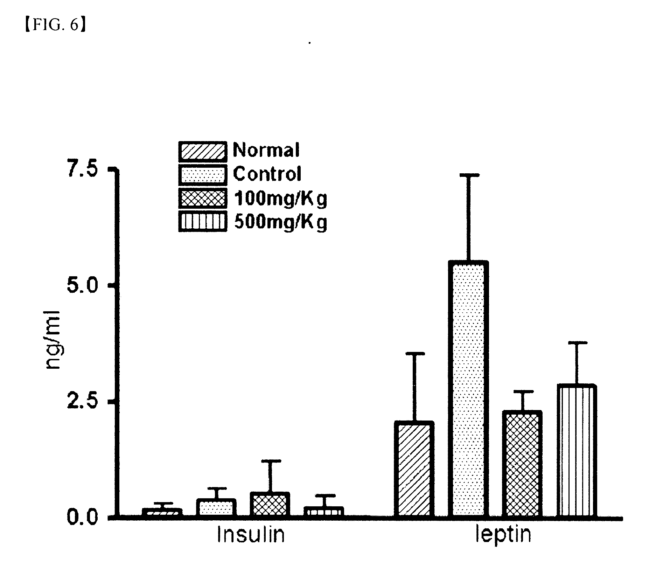

FIG. 6 is a graph illustrating the contents of leptin and insulin in blood in an ethanol extract diet group of the purple-colored potato.

### DETAILED DESCRIPTION OF THE PREFERRED EMBODIMENT

Hereinafter, preferable embodiments according to the present invention will be described with reference to the accompanying drawings.

In the present invention, the extract of purple-colored potato *(Solanum tuberosum* L. cv. Bora valley) having an obesity-suppressing activity means that it includes a crude extract of purple-colored potato extracted by adding to water or alcohol to the purple-colored potato.

The purple-colored potato extract is manufactured by adding water to the purple-colored potato to perform a hot water extraction, or adding alcohol to perform a cold dipping or low-temperature heating process, and also may be manufactured in the form of powder. Preferably, the alcohol added to extract the extract of purple-colored potato may be used from 5 to 100 % ethanol or methanol, and more preferably 70% ethanol.

In the present invention, the purple-colored potato is extracted about 3 times with ethanol, concentrated under a reduced pressure, and then freeze-dried to obtain its powder, which will be used later.

The ethanol extract of purple-colored potato according to the present invention has an obesity-suppressing effects such as a fat-control activity of adipose cells to significantly reduce a fat content in the procedure in which preadipocyte is modified into adipocyte; the suppression of formation of new blood vessels and tubes which are differentiated into adipose fats; and the suppression of growth of the adipose fat.

Also, the present invention includes the suppression of differentiation into adipose cells and expression of proteins inducing obesity by means of the ethanol extract of novel purple-colored potato *(Solanum tuberosum* L., cv. Bora valley) registered as the national potato variety in Korea, rather than a genetically modified organism (GMO).

And, in the present invention, various foods may be manufactured using the conventionally known methods by adding the ethanol extract of purple-colored potato to general favorite foods, namely noodles such as Ramen and wet noodle, bean curd, cereal, breads, chewing gum, candies, snacks, and the ethanol extract of purple-colored potato may also be formulated into conventional formulations such as tablet, granule, pill, hard capsule, soft capsule or solution, and formulated into raw juice, pouch, beverage or tea. The other ingredients rather than the above-mentioned ingredient may be suitably mixed with the formulations by those skilled in the art. The healthy and functional food for treating obesity patients according to the present invention may be manufactured as a major/minor ingredient of foods made of the extract of purple-colored potato *(Solanum tuberosum* L., cv. Bora valley), or manufactured by adding the extract of purple-colored potato *(Solanum tuberosum* L., cv. Bora valley) to other foods. At this time, sitologically available food-aiding additives may be used herein.

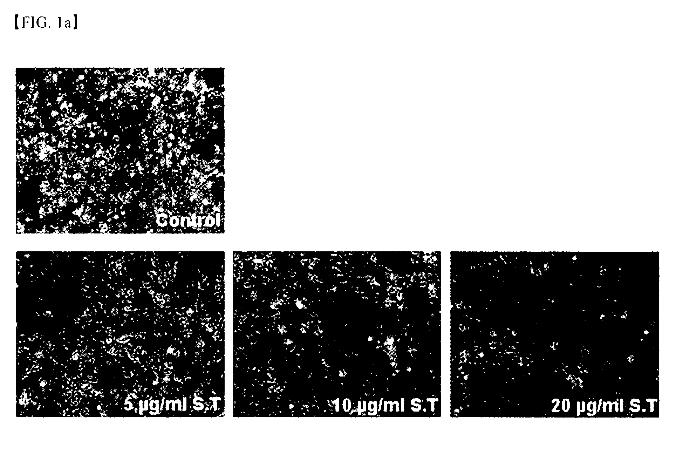

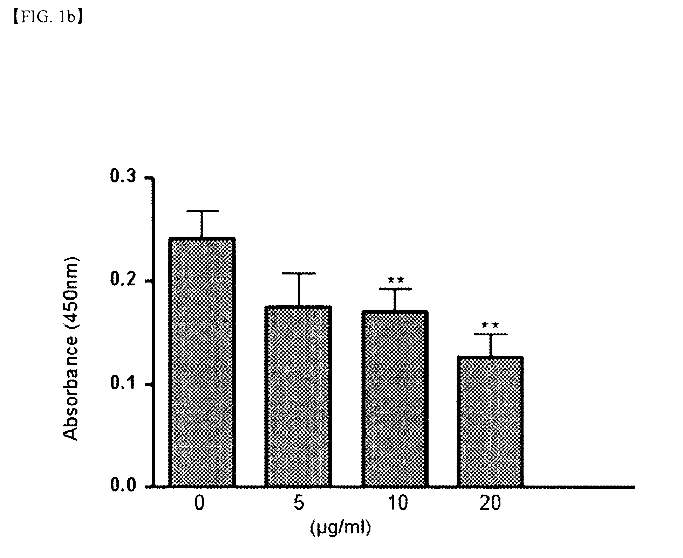

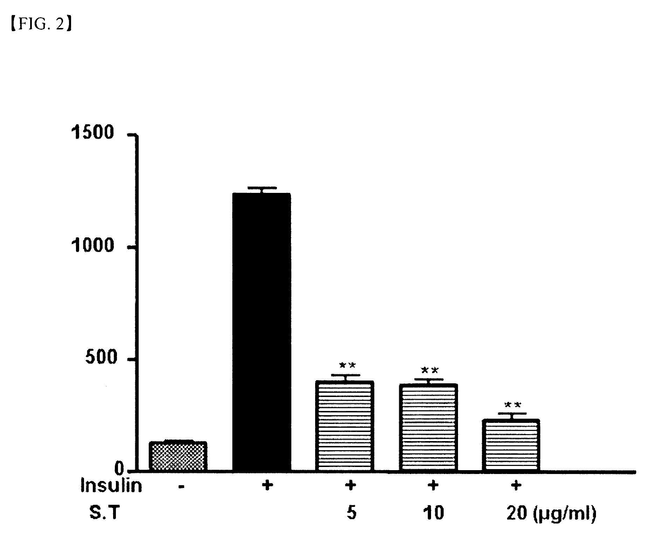

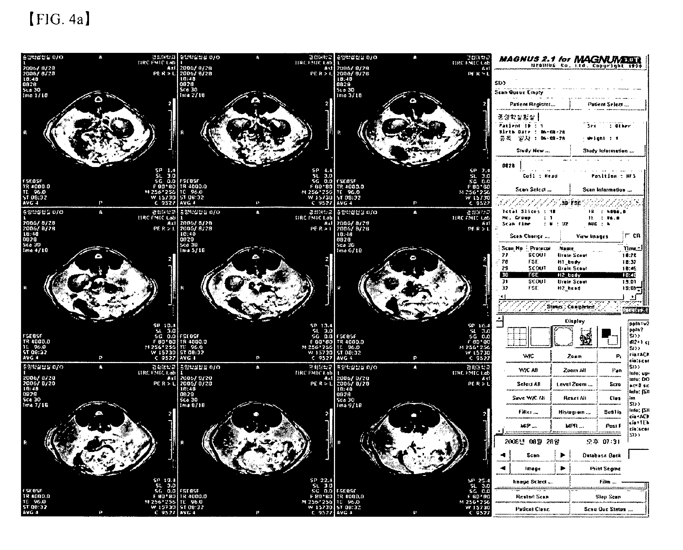

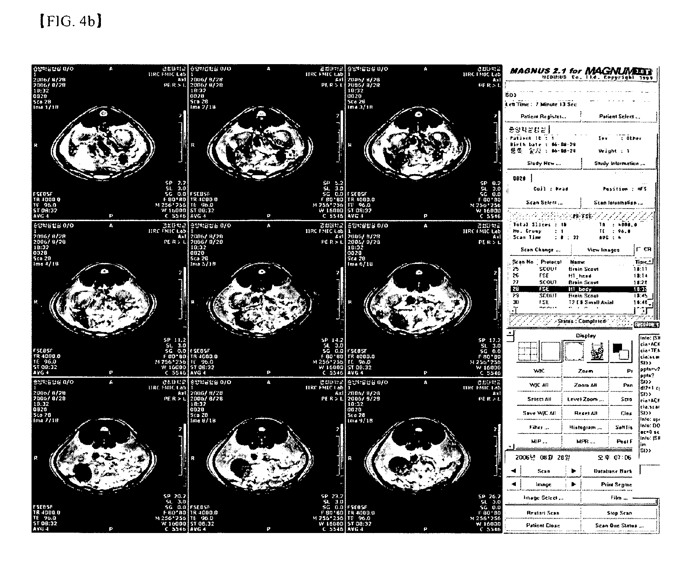

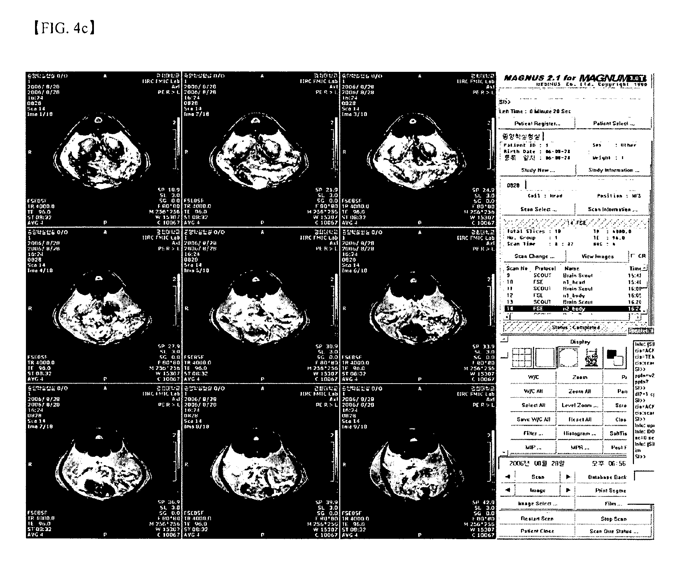

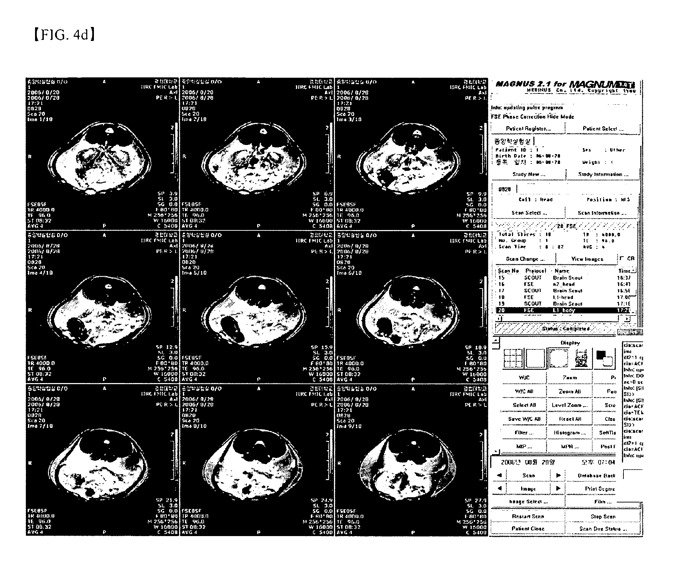

The extract of purple-colored potato *(Solanum tuberosum* L., cv. Bora valley) of the present invention was administered into rats to measure lipid accumulated in the adipose cells (Oil Red staining method, absorbance method). As a result, it was revealed that the extract of purple-colored potato has a significant effect on the suppression of the lipid (FIG. 1a and FIG. 1b). Also, the healthy and functional food containing the ethanol extract of purple-colored potato according to the present invention may be effectively used to treat obesity patients since the ethanol extract of purple-colored potato also significantly reduce a content of leptin in a concentration-dependent manner (FIG. 2). In particular, in the present invention, it was seen that, when the dietary fat and the purple-colored potato ethanol fraction were administered into laboratory animals to measure a level of abdominal visceral obesity using a magnetic resonance imaging system. As a result, it was revealed that the abdominal visceral obesity of a diet mouse group administered with the purple-colored potato ethanol fraction is significantly reduced, compared to that of a mouse group administered with the high fat diet, as shown in FIG. 4a to FIG. 4d, indicating that the purple-colored potato ethanol fraction may be very effectively used to treat abdominal obesity patients.

Accordingly, the extract of purple-colored potato (*Solanum tuberosum* L*.,* cv. Bora valley) according to the present invention, which is a novel subject matter for novel healthy and functional foods for treating obesity patients, may be effectively used within a wide age group of teenagers and adults and old persons, and therefore may be used to develop a healthy and functional food for preventing and reducing obesity.

The extract of purple-colored potato *(Solanum tuberosum* L., cv. Bora valley) according to the present invention is suitable to be used as food additives from the results of the acute toxicity and subacute toxicity tests.

### Productive example 1. Production of Purple-colored Potato Ethanol Extract

37.8 Kg of purple-colored potato was extracted with 63 L of ethanol three times, concentrated under a reduce pressure in a rotary vacuum distiller (Buchi 461), and then freeze-dried to obtain 366 g of its powder (yield: 0.97%).

### Productive example 2. Production of Solution

The ethanol extract powder of purple-colored potato prepared in Productive example 1 was dissolved in an emulsifying agent (DMSO) to prepare a solution.

### Productive example 3. Production of Raw Juice

50 Kg of the purple-colored potato was pressed with a compressor to obtain 714 g of a purple-colored potato raw juice (yield: 1.43%).

### Experimental example 1

### 1. In vivo Induction of Mouse from Pre-obesity Cells into Adipose Cells

A fat cell line (STS-L1) was divided into a 6-well plate at a density of 10⁶ cells and grown for at least one day until the well was full of the cells. Then, 0.5 mM isobutylmethylxanthine, 1µM dexamethasone and 1 µg/Mℓ insulin were mixed with a DMEM culture solution (Well-gene) and the resultant mixture was added to the cells. The cells was induced for their differentiation for two days, and only insulin was added to the DMEM culture solution to be maintained as the adipose cells when the cells were changed in shape.

### 2. Measurement of Lipid Accumulated in Adipose Cells

### A. Oil Red Staining Method

The adipose cells were stained with Oil Red O and their absorbance was measured to confirm a suppressive effect of lipid according to the present invention. The Oil Red staining method, which is a method for staining only lipid in the cells with a red color, may judge the suppression of accumulation of the lipid due to the obesity. FIG. 1a shows that an adipose cell line STS-L1 of a mouse was treated with insulin and dexamethasone to induce obesity, treated with the ethanol extract of purple-colored potato at an increasing concentration, fixed with formaldehyde for 72 hours and measured for a level of the suppression of production of lipid in the adipose cells using an Oil Red O staining method. As shown in FIG. 1a, it was confirmed that the whole cells were tinged with red color since the positive control has a high lipid content, whereas the adipose cells in the culture solution supplemented with the ethanol extract of purple-colored potato were hardly stained due to the low lipid content.

### B. Absorbance Method

Also, the DMEM culture solution was removed off, and the cell pellet was mildly washed with phosphate saline, and 1 Mℓ of the Oil Red staining solution was added to each of the wells. If the staining is completed, the cell pellet was washed again with phosphate saline and then photographed to judge a staining level of the adipose cells. Then, the adipose cells was dissolved in 400 µℓ of isopropanol, put into a 96 well plate at a quantity of 100 µℓ, and then their absorbance was measured at wavelength of 450 nm. It was observed that the more darkly stained adipose cells have a relatively high absorbance value. In order to qunatitify a content of lipid, the adipose cells were dissolved in isopropanol to qunatitify a content of lipid in the cells. As a result, it was revealed that the ethanol extract powder of purple-colored potato has an effective lipid-suppressing activity since the absorbance of the adipose cells is lowered in a concentration-dependent manner, as shown in FIG. 1b.

### 3. Measurement of Leptin Content in Adipose Cells

The supernatant of the treated adipose cell line was separated and centrifuged at a rotary speed of 13,000 rpm for 1 minute to obtain a clean supernatant. The resultant clean supernatant was stored at -80 °C for one day, and then a content of leptin was measured using an enzyme-linked immunosorbent assay (ELISA), and the results are shown in FIG. 2 (a unit of concentration is by pg/Mℓ).

As shown in FIG. 2, it was seen that the healthy and functional food containing the ethanol extract of purple-colored potato according to the present invention may be useful to treat obesity patients since the content of leptin is also significantly lowered in a concentration-dependent manner.

### Experimental example 2

### 1. Breeding of Laboratory Animals and Collection of Test Samples

Laboratory animals were kindly provided from Daehan Biolink Co. Ltd., and sufficiently adapted and bred for about 2 weeks under animal keeper's constant conditions (temperature: 20±2 °C, humidity: 40-60 %, brightness: 12 hour light/dark cycle). The female Sprague-Dawley rat (body weight: 130-150 g) was used herein, and divided into two groups: a normal diet group and a high fat diet group. Four 4 weeks-old rats was sacrificed before rats were supplied with experimental diet, and used as a reference animal. The experimental diet was listed in the following Table 1. Here, the normal diet group was supplied with fat that accounts for 11.7% of the total dietary calory as an AIN 76A diet, and the high fat diet group was supplied with fat that accounts for 40 % of the total calory by employing a beef tallow as a fat source. 4 week-old laboratory animals whose weaning period was finished were supplied with the experimental diet. In order to observe the changes during a growth period and after the growth period for 10-week breeding period, difference between the normal diet group and the high fat diet group was observed at time points: 6, 8 and 10 weeks old where the rats was supplied with the experimental diet for 2, 4 and 6 weeks, respectively. 100 and 200 mg/kg of 2 % methylcellulose where the ethanol fraction of purple-colored potato is dissolved in saline were orally administered into the two groups of the laboratory animals for 10 weeks, respectively.

The laboratory animals were dividedly bred by two, and supplied with water and diet without any of limitations. During the experiment period, the dietary intake and body weight were measured two times per week. A food efficiency ratio (FER) was measured for a period from the staring day of experimental diet to a day when the rate were sacrificed, and calculated by dividing a body weight gain during the experiment period by the dietary intake during the experiment period. The rats were bred with the experimental diet during a period from 4 weeks old for 12 weeks, and then the 6, 8 and 10-week old rats were randomly selected by four from each of the experimental groups and their bloods and organs were extracted. Intercellular brown adipose fat and visceral fat were separated and their weights were measured, and frozen with liquid nitrogen right after the extraction. The bloods were collected using a heart puncture method when the rats were sacrificed, and then centrifuged at a rotary speed of 3,000 rpm for 15 minutes to obtain serum, which was stored at -70 °C for the future use for analysis.

### 2. Collection and Assay of Test Samples

### A. Collection of Test Samples

The 4, 6, 8 and 10-week old rats were randomly selected by four from each of the experimental groups and their bloods and organs were extracted. Intercellular brown adipose fat, visceral fat, a kidney and a liver were separated and their weights were measured, and frozen with liquid nitrogen right after the extraction. The bloods were collected using a heart puncture method when the rats were sacrificed, and then centrifuged at a rotary speed of 3,000 rpm for 15 minutes to obtain serum, which was stored at -70°C for the future use for analysis.

### B. Abdominal Fat Distribution Analysis using MRI

A day ago when the 16-week old laboratory animals were sacrificed, their abdomens were photographed using a magnetic resonance imaging system to observe body fat distributions in the abdomens.

### C. Lipid Content Analysis in Blood

Total contents of cholesterol, HDL cholesterol and triglyceride in serum were measured under the entrustment of the Seoul Clinical Laboratories.

### D. Leptin and Insulin Content Analysis in Blood

Contents of leptin and insulin in serum were measured under the entrustment of the Seoul Clinical Laboratories.

### E. Evaluation of Data

Significance in the averages between the normal diet group and the high fat diet group was tested using a Student's t-test, and the changes according to the week ages was tested using a 1-way ANOVA, and then a Duncan's multiple range test was carried out at a level of α= 0.05. Also, a level of the dietary fat and effect factors according to the week ages were analyzed using a 2-way ANOVA. The significance was tested at a levels of α=0.05 and α= 0.01, and all statistical analyses were applied to the SAS program. The results were represented by mean ± standard deviation.

**Table 1**

| Diet Composition (g/Kg) | | |
|---|---|---|
| Ingredients | Normal Diet Group | High Fat Diet Group |
| Casein | 200 | 200 |
| DL-methionine | 3 | 3 |
| Corn starch | 150 | 150 |
| Sucrose | 500 | 345 |
| Cellulose | 50 | 50 |
| Corn oil | 50 | - |
| Beer Tallow | - | 205 |
| Salt mixture | 35 | 35 |
| Vitamin mixture | 10 | 10 |
| Choline bitartrate | 2 | 2 |
| Fat % (calory) | 11.7 | 40.0 |

| | | |
|---|---|---|
| 1) Normal diet group: AIN-76A (Dyets Inc., Bethlehem, PA USA) 2) High fat diet group: AIN-76 (Dyets Inc., Bethlehem, PA USA) | | |

### 3. Results

### A. Dietary Intake, Body Weight Gain and Food Efficiency Ratio

The results of dietary intake, body weight gain and food efficiency ratio in the measured laboratory animals are listed in the following Table 2. There is no significant difference in the dietary intakes between the normal diet group and the high fat diet group, but the body weight gain was significantly higher due to the different level of fat intake in the high fat diet group than the normal diet group (P< 0.05) when it was measured 2 weeks and 12 weeks after the supply of the experimental diet. Also, the food efficiency ratio was higher due to the different level of dietary fat in the high fat diet group fed with the experimental diet having a high energy density, and the food efficiency ratio was significantly higher in the high fat diet group of the 16-week old rats fed with the experimental diet for 12 weeks as the difference in the food efficiency ratio between the two groups increases with continuous supply of the high fat diet (P<0.01). There was not difference in the dietary intake between the two experimental groups, and the body weight gain and the food efficiency ratio were higher in the high fat diet group than the normal diet group, but there was statistical significance in the effect on the purple-colored potato ethanol fraction.

**Table 2**

| Food Efficiency Ratio | | | |
|---|---|---|---|
| | Dietary Intake | Weight Gain (g/day) | Food Efficiency Ratio |
| Normal diet group | 30.18±4.82 | 6.82±3.29 | 0.54±0.31 |
| High fat diet group | 36.19±4.01 | 11.38±2.91 | 0.98±0.10 |
| High fat diet group + Purple-colored potato + Ethanol Fraction 100mg/Kg | 35.89±3.88 | 10.25±2.67 | 0.89±0.22 |
| High fat diet group + Purple-colored potato + Ethanol Fraction 200mg/Kg | 34.41±3.79 | 8.29±3.31 | 0.86±0.48 |
| p-value | 0.1531 | 0.1294 | 0.1542 |

### B. Weight of Adipose Fat

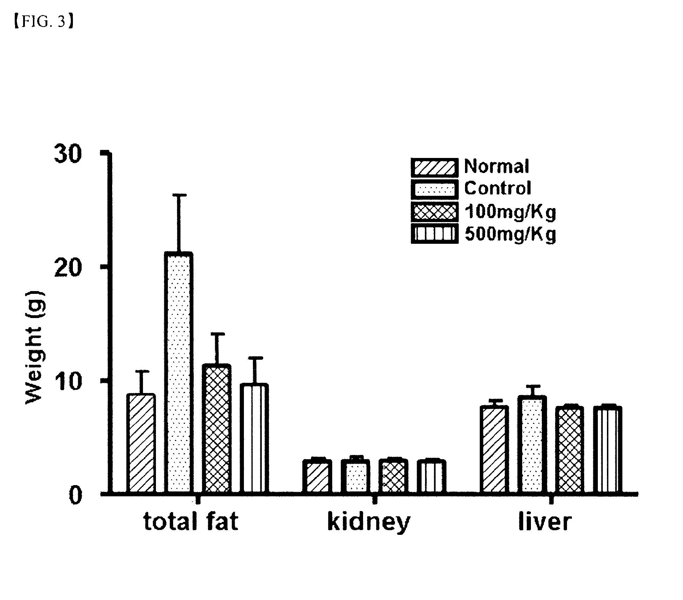

The weights of intercellular brown adipose fat and visceral fat by the supply of the dietary fat and the purple-colored potato ethanol fraction were compared with each other. As shown in FIG. 3, it was revealed that the weight of the brown adipose fat was higher in the rats fed with the high fat diet than the rats fed with the normal diet, indicating that the higher weight of the brown adipose fat in the high fat diet group was lowered by the dietary supply of the purple-colored potato ethanol fraction.

### C. Photographing of Abdomen by Magnetic Resonance Imaging System

A level of the abdominal visceral fat in the supply of the dietary fat and the purple-colored potato ethanol fraction was photographed using a magnetic resonance imaging system. As shown in FIG. 4a to FIG. 4d, it was revealed that the level of the abdominal visceral fat was significantly lower in the rats fed with the high fat diet than the rats fed with the purple-colored potato ethanol fraction

### D. Lipid Content in Blood

There was not difference in the total cholesterol content in blood between the experimental groups, but the high-density lipoprotein (HDL) cholesterol was lower in the high fat control than in the normal control, and significantly higher in the diet group fed with the purple-colored potato ethanol fraction than the high fat control. The triglyceride in blood was significantly higher in the high fat control than the normal control, whereas the triglyceride in blood was significantly lower in the diet group fed with the purple-colored potato ethanol fraction, which was about 50 % of the high fat control.

### E. Leptin and Insulin content in Blood

The leptin content in blood was significantly lower in the purple-colored potato ethanol fraction than the high fat control, which is substantially identical to the normal control. The insulin content in blood was also lower in the diet group of the purple-colored potato ethanol fraction, which is substantially identical to the normal control.

### Experimental example 3. Subacute Toxicity Test

### A. Materials and Laboratory Animals

The solutions prepared in Productive example 2 was used as the experimental materials, and the laboratory animals were kindly provided from Daehan Biolink Co. Ltd., and sufficiently adapted and bred for about 2 weeks under animal keeper's constant conditions (temperature: 20±2 °C, humidity: 40-60 %, brightness: 12 hour light/dark cycle). 10 female Sprague-Dawley rats (body weight: 130-150 g) were prepared.

### B. Procedure

The solutions were orally administered to laboratory animals at 2 g/kg bw/day for 2 weeks to measure the changes in rat body weights.

### C. Test Results

There was no change within the range of the mean body weight gain of the laboratory animals, which remained alive.

Through such safety test, it was considered that the purple-colored potato extract of the present invention did not adversely affect the human body.

Hereinafter, a healthy and functional food for treating obesity patients was manufactured, and also tea and beverage may be manufactured by adding the extract of purple-colored potato *(Solanum tuberosum* L., cv Bora valley) according to the present invention to a variety of solid foods including a chewing gum, noodles such as Ramen, candies, snacks, cereal, breads, etc.

### Example 1. Production of Biscuit

7 % by weight of the extract of purple-colored potato *(Solanum tuberosum* L., cv Bora valley) prepared in Productive example 1 was mixed with 19.59 % by weight of cake gluten flour grade 1, 22.22 % by weight of bake gluten flour grade 1, 4.80 % by weight of refined sugar, 0.73 % by weight of table salt, 0.78 % by weight of glucose, 11.15 % by weight of palm shortening, 1.54 % by weight of ammonium, 0.17 % by weight of sodium bicarbonate, 0.16 % by weight of sodium bisulphite, 1.45 % by weight of rice flour, 0.0001 % by weight of vitamin B1, 0.0001 % by weight of vitamin B2, 0.04 % by weight of milk perfume, 21.3298 % by weight of water, 1.16 % by weight of evaporated dry milk, 0.29 % by weight of milk replacer, 0.03 % by weight of calcium phosphate monobasic, 0.29 % by weight of sprayed salt and 7.27 % by weight of sprayed milk, and the resultant mixture was then subject to the conventional method to prepare a Biscuit.

### Example 2. Production of Chewing Gum

7 % by weight of the extract of purple-colored potato *(Solanum tuberosum* L., cv Bora valley) prepared in Productive example 1 was mixed with 20 % by weight of gum base, 70 % by weight of sugar, 1 % by weight of spices and 2 % by weight of water, and the resultant mixture was then subject to the conventional method to prepare a chewing gum.

### Example 3. Production of Candy

10 % by weight of the extract of purple-colored potato *(Solanum tuberosum* L., cv Bora valley) prepared in Productive example 1 was mixed with 50 % by weight of sugar, 39.8 % by weight of starch syrup and 0.2 % by weight of spices, and the resultant mixture was then subject to the conventional method to prepare a candy.

### Example 4. Production of Beverage

10 % by weight of the extract of purple-colored potato *(Solanum tuberosum* L., cv Bora valley) prepared in Productive example 1 was mixed with 0.26 % by weight of honey, 0.0002 % by weight of thioctamide, 0.0004 % by weight of nicotinamide, 0.0001 % by weight of riboflavin sodium hydrochloride, 0.0001 % by weight of pyridoxine hydrochloride, 0.001 % by weight of inositol, 0.002 % by weight of orotic acid and 89.7362 % by weight of water, and the resultant mixture was then subject to the conventional method to prepare a beverage.

### Example 5. Production of Tablet

In order to formulate a tablet, 200 mg of spray-dried powder of the extract of purple-colored potato *(Solanum tuberosum* L., cv Bora valley) prepared in Productive example 1, 15 mg of vitamin B₁, 15 mg of vitamin B₂, 25 mg of vitamin C, 25 mg of vitamin B₆ and 15 mg of nicotinamide were mixed, and 200 mg of lactose powder, 25 mg of sodium silicoaluminate, 25 mg of sucrose fatty acid ester, 25 mg of hydroxypropylmethyl cellulose, 15 mg of glycerin fatty acid ester, 15 mg of zinc oxide and 15 mg of β-cyclodextrin was added and mixed to prepare a tablet with a tablet making machine.

### Example 6. Production of Hard Capsule

100 mg of the extract of purple-colored potato *(Solanum tuberosum* L., cv Bora valley) prepared in Productive example 1, 15 mg of vitamin B₁. 15 mg of vitamin B₂, 25 mg of vitamin C, 25 mg of vitamin B₆, 15 mg of nicotinamide, 25 mg of citric acid, and 400 mg of lactose were added and mixed with 500 mg of purified water. Then, the mixture was put into a granulator and molded into a granule shape, and the molded granule was dried at 40~50 °C in a hot-air dryer, and then passed through a 12-14 mesh sieve to obtain a uniform granule, with which a hard capsule was charged.

The description proposed herein is just a preferable example for the purpose of illustrations only, not intended to limit the scope of the invention, so it should be understood that other equivalents and modifications could be made thereto without departing from the spirit and scope of the invention as apparent to those skilled in the art. Therefore, it should be understood that the present invention might be not defined within the scope of which is described in detailed description but within the scope of which is defined in the claims and their equivalents.

As described above, it was revealed that the ethanol extract of purple-colored potato *(Solanum tuberosum* L., cv Bora valley) according to the present invention suppresses the differentiation into adipose cells since the ethanol extract lowers a lipid content of the adipose cells and reduce an amount of the expressed leptin, and it was also seen that the ethanol extract of purple-colored potato *(Solanum tuberosum* L., cv Bora valley) according to the present invention has effective activities of significantly reducing a content of triglyceride and cholesterol in a concentration-dependent manner when the content of triglyceride and cholesterol was measured after administered with a high fat diet and of increasing high-density lipoproteins (HDL) in blood.

Accordingly, the ethanol extract of purple-colored potato *(Solanum tuberosum* L., cv Bora valley) according to the present invention may be effectively used as a healthy and functional food for treating obesity patients.

## Claims

1. A healthy and functional food having an obesity-suppressing activity, the food containing a purple-colored potato extract extracted by adding water or aqueous alcohol solution to a purple-colored potato *(Solanum tuberosum* L. cv. Bora valley).

2. The healthy and functional food having an obesity-suppressing activity according to claim 1, wherein the purple-colored potato extract is extracted by adding 5 to 100% aqueous ethanol solution to purple-colored potato.

3. The healthy and functional food having an obesity-suppressing activity according to claim 1, wherein the healthy and functional food is selected from the group consisting of tablet, granule, pill, hard capsule, soft capsule and solution formulations.

4. The healthy and functional food having an obesity-suppressing activity according to claim 1, wherein the healthy and functional food is selected from the group consisting of a raw juice, a wine, a pouch, a power, a beverage and a green tea.

5. A food additive having an obesity-suppressing activity as an extract of purple-colored potato *(Solanum tuberosum* L. cv. Bora valley).

6. A healthy and functional food for treating obesity patients, the food containing the food additive as an extract of purple-colored potato *(Solanum tuberosum* L. cv. Bora valley) as defined in claim 5 in a favorite food selected from the group consisting of a chewing gum, candies and snacks.

7. A healthy and functional food for treating obesity patients, the food containing the food additive as an extract of purple-colored potato *(Solanum tuberosum* L. cv. Bora valley) as defined in claim 5 in a food selected from the group consisting of noodles such as Ramen and wet noodle, bean curd, raw-eating power mixture, and cereal and breads.
